# EUROPEAN PATENT SPECIFICATION

(11) **EP 3 401 390 B1**
(45) Date of publication and mention of the grant of the patent: **19.08.2020**
(21) Application number: 17788678.5
(22) Date of filing: 18.04.2017
(51) Int. Cl.: C12N 1/21, C12P 13/08, C12N 15/90, C12R 1/15

(54) **METHOD FOR CONSTRUCTING ENGINEERED CORYNEBACTERIUM GLUTAMICUM BACTERIA HAVING HIGH PRODUCTION OF L-LYSINE**
VERFAHREN ZUR HERSTELLUNG MANIPULIERTER CORYNEBACTERIUM-GLUTAMICUM-BAKTERIEN MIT HOHER PRODUKTION VON L-LYSIN
PROCÉDÉ DE CONSTRUCTION DE BACTÉRIES CORYNEBACTERIUM GLUTAMICUM SYNTHÉTIQUES PRODUISANT UN TAUX ÉLEVÉ DE L-LYSINE

(30) Priority: 27.04.2016 CN 201610270794
(43) Date of publication of application: 14.11.2018
(73) Proprietor: Qilu University Of Technology, Jinan, Shandong 250353 (CN); Zhucheng Dongxiao Biotechnology Co., Ltd., Weifang, Shandong 262200 (CN); Shandong Provincial Feed Quality Inspection Institute, Jinan, Shandong 250022 (CN)
(72) Inventor: WANG, Ruiming, Jinan Shandong 250353 (CN); WANG, Junqing, Jinan Shandong 250353 (CN); WANG, Lei, Jinan Shandong 250353 (CN); WANG, Tengfei, Jinan Shandong 250353 (CN); LI, Guihua, Jinan Shandong 250022 (CN); WANG, Jianbin, Weifang Shandong 262200 (CN); SUI, Songsen, Weifang Shandong 262200 (CN)
(74) Representative: Sun, Yiming
(86) International application number: PCT/CN2017/080902
(87) International publication number: WO 2017/186026

(56) References cited:
- EP-A2- 2 803 721
- WO-A1-2015/092599
- CN-A- 101 600 796
- CN-A- 101 605 886
- CN-A- 103 282 486
- CN-A- 105 950 524
- KR-A- 20150 023 598
- JIANG YAN-ZHE ET AL: "Effects of NCgl1221 Knockout and pyc Overexpression on Glutamine Fermentation", XIAN DAI SHI PIN KE JI = MODERN FOOD SCIENCE AND TECHNOLOGY, HUANAN LIGONG DAXUE,SOUTH CHINA UNVERSITY OF TECHNOLOGY, CN, vol. 30, no. 10, 31 December 2014 (2014-12-31), pages 96-101, XP009508135, ISSN: 1673-9078 [retrieved on 2014-09-24]
- AHMED ZAHOOR ET AL: "METABOLIC ENGINEERING OF CORYNEBACTERIUM GLUTAMICUM AIMED AT ALTERNATIVE CARBON SOURCES AND NEW PRODUCTS", COMPUTATIONAL AND STRUCTURAL BIOTECHNOLOGY JOURNAL, vol. 3, no. 4, 1 October 2012 (2012-10-01) , pages 1-11, XP55203417, ISSN: 2001-0370, DOI: 10.5936/csbj.201210004
- JIANG, YANZHE ET AL.: 'Effects of NCgl 1221 Knockout and pyc Overpression on Glutamine Fermentation' MODERN FOOD SCIENCE & TECHNOLOGY vol. 30, no. 10, 31 December 2014, pages 96 - 101, XP009508135
- JUN NAKAMURA ET AL.: 'Mutations of the Corynebacterium glutamicum NCg11221 Gene , Encoding a Mechanosensitive Channel Homolog, Induce 1-Glutamic Acid Production' APPLIED AND ENVIROMENTAL MICROBIOLOGY vol. 14, no. 73, 31 July 2007, ISSN 0099-2240 pages 4491 - 4498, XP002691368
- CHIKAKO YAMASHITAE ET AL.: 'L-Glutamate secretion by the N-terminal domain of the Corynebacterim glutamicum NCg11221 mechanosensitive channel' BIOSCIENCE. BIOTEHNOLOGY . BIOCHEMISTRY vol. 77, no. 5, 07 May 2013, ISSN 1347-6947 pages 1008 - 1013, XP055436377

### SEQUENCE LISTING

<110> Qilu University of Technology Zhucheng Dongxiao Biotechnology Co., Ltd. Shangdong Provincial Feed Quality Inspection Institute
<120> A method for Constructing Engineered Corynebacterium Glutamicum
   Bacteria Having a High Production of L-lysine
<160> 5
<170> PatentIn version 3.5
<210> 1
   <211> 1602
   <212> DNA
   <213> Corynebacterium glutamicum
<400> 1
   atgattttag gcgtacccat tcaatatttg ctctattcat tgtggaattg gattgtcgat      60
   accggttttg atgtagcaat tatcctggtc ttggcgtttt tgattccacg tatcggccga     120
   ctggccatgc gtattatcaa gcgccgagtg gagtctgcag ccgatgcgga caccactaag     180
   aaccagctcg cgttcgccgg cgttggcgtt tatatcgcgc aaattgtggc gtttttcatg     240
   cttgccgtct ccgcgatgca ggcttttggt ttctctctcg cgggcgctgc gattccggca     300
   accattgcgt cagctgccat tggccttggt gcgcagtcga ttgttgcgga cttcttggcc     360
   ggatttttca tcctgacgga aaagcaattc ggcgtgggtg actgggtgcg ttttgagggc     420
   aacggcatcg ttgtcgaagg caccgtcatt gagatcacca tgcgcgcgac caaaattcgc     480
   acgattgcac aagagaccgt gatcatcccc aactccacgg cgaaagtgtg catcaacaat     540
   tctaataact ggtcgcgtgc ggttgtcgtt attccgatcc ccatgttggg ttctgaaaac     600
   atcacagatg tcatcgcgcg ctctgaagct gcgactcgtc gcgcacttgg ccaggagaaa     660
   atcgcaccgg aaatcctcgg tgaactcgat gtgcacccag ccacggaagt cacgccgcca     720
   acggtggtcg gcatgccgtg gatggtcacc atgcgtttcc tcgtgcaagt caccgccggc     780
   aatcaatggc tggtcgaacg cgccatccgc acagaaatca tcagcgaatt ctgggaagaa     840
   tacggcagcg caaccactac atcgggaacc ctcattgatt ccttacacgt tgagcatgaa     900
   gagccaaaga cctcgcttat cgacgcctcc ccccaggctc ttaaggaacc gaagccggag     960
   gctgcggcga cggttgcatc gctagctgca tcctctaacg acgatgcaga caatgcagac    1020
   gcctcggtga tcaatgcagg caatccagag aaggaacttg attccgatgt gctggaacaa    1080
   gaactctcca gcgaagaacc ggaagaaaca gcaaaaccag atcactctct ccgaggcttc    1140
   ttccgcactg attactaccc aaatcggtgg cagaagatcc tgtcgtttgg cggacgtgtc    1200
   cgcatgagca cgtccctgtt gttgggtgcg ctgctcttgc tgtcactatt taaggtcatg    1260
   actgtggaac caagtgagaa ttggcaaaac tccagtggat ggctgtcacc aagcactgcc    1320
   acctcaactg cggtgaccac ctccgaaact tccgcgccag taagcacgcc ttcgatgaca    1380
   gtgcccacta cggtggagga gaccccaacg atggaatcta acgtcgaaac gcagcaggaa    1440
   acctcaaccc ctgcaaccgc aacgccccag cgagccgaca ccatcgaacc gaccgaggaa    1500
   gccacgtcgc aggaggaaac gactgcgtcg cagacgcagt ctccagcagt ggaagcacca    1560
   accgcggtcc aagagacagt tgcgccgacg tccacccctt ag                       1602
<210> 2
   <211> 29
   <212> DNA
   <213> Synthetic
<400> 2
   cgggatcccg tttttcatgc ttgccgtct 29
<210> 3
   <211> 34
   <212> DNA
   <213> Synthetic
<400> 3
   aaggccagca aaagctaaag cccaagaatg caac 34
<210> 4
   <211> 32
   <212> DNA
   <213> Synthetic
<400> 4
   ggtggtcggc atttttgctg gccttttgct ca 32
<210> 5
   <211> 34
   <212> DNA
   <213> Synthetic
<400> 5
   cataatcggc tggatcctag tgactggcga tgct 34

## Description

### FIELD OF THE INVENTION

The present invention relates to an application of engineered Corynebacterium glutamicum bacteria with a high production of L-lysine and belongs to the biotechnology field.

### BACKGROUND OF THE INVENTION

L-lysine, an aspartic acid, is one of the eight essential amino acids in human bodies and animals. With many physiological functions, such as balancing amino acids, regulating metabolic balance in the body, increasing the absorption and utilization rate of the body to cereal proteins and promoting the growth and development of the body, L-lysine is widely applied in the feeds, foods and medicine industries. As a result of the great demand on the market, L-lysine has become the second largest amino acid produced after glutamic acid and its annual production is around 3.5 million tons at present. Three main methods are used to produce L-lysine in the industry, i.e., proteolysis, chemical synthesis and microbial fermentation. The microbial fermentation is a mainstream method used to produce L-lysine at present for its advantages such as low cost, high productivity and low pollution.

Now, the main microorganism for producing L-lysine is bacteria including Corynebacteria, Brevibacteria, Candida, Pseudomonas, Bacilli and Escherichia, among which the Corynebacterium glutamicum in the genus Corynebacteria is the most widely applied. Microorganisms in nature can produce L-lysine directly through fermentation, but the productivity is relatively low, so it is necessary to modify the microorganisms. For example, Chinese Patent CN1539015 discloses multiple improved gene loci which can be used to increase the production of lysine, including accBC, accDA, catA, cysD and cysE. Chinese patent literature CN101600796 (application number 200780048626.8), CN1974760 (application number 200610163142.5) and CN103243042 (application number 201310092638.8) inactivate the genes NCg22534, NCgl1835 and NCg21090 respectively and obtain a series of engineered Corynebacterium glutamicum bacteria with a high production of L-lysine.

WO 2015/092599 A1 discloses an improved method for the fermentative production of gamma-aminobutyric acid (GABA) by cultivating a recombinant microorganism expressing an enzyme having a glutamate decarboxylase activity, wherein the recombinant microorganism has an amended expression of certain enzymes influencing the GABA production and / yield. Also disclosed are corresponding recombinant hosts, recombinant vectors, expression cassettes and nucleic acids suitable for preparing such hosts as well as to a method for preparing pyrrolidone and polymers making use of GABA as obtained by fermentative production.

KR 2015 0023598 A discloses a Corynebacterium sp. microorganism with enhanced L-arginine productivity by improved activity of aspartate ammonialyase and/or aspartate amino transferase, and a method for producing L-arginine by using the Corynebacterium sp. microorganism.

EP 2 803 721 A2 discloses a recombinant microorganism having enhanced ability of producing putrescine at high yield, which is generated by weakening the activity of NCgl1469 in a microorganism of Corynebacterium genus that is modified to produce putrescine, and a method for producing putrescine using the same.

Jiang Yan-Zhe et al. have knocked out *NCgl1221* of the glutamine-producing strain GM34 using homologous recombination technology, resulting in the construction of the mutant strain GM34:\NCgl 1221 in order to reduce glutamic acid accumulation during glutamine fermentation and to improve the yield of glutamine. *Pyc* encoding pyruvate carboxylase was also overexpressed in the constructed strain GM34-pX MJ 19pyc. Fermentation carried out in 5-L bottles showed that *NCgl1221* knockout reduced glutamic acid accumulation by 19.05%. Overexpression of *pyc* resulted in a promotion of glutamine production and conversion rate by 5.54% and 2.37%, respectively.

### SUMMARY OF THE INVENTION

In order to overcome the shortcomings of the prior art, a new application of an engineered Corynebacterium glutamicum bacteria with a high production of L-lysine is provided. The present invention is obtained by modifying Corynebacterium glutamicum bacterium strains, with the specific strategy as below: the endogenous gene NCgl1221 (encoding the protein for transporting glutamic acid out of cells) of Corynebacterium glutamicum is inactivated to reduce the extracellular secretion of glutamic acid during lysine fermentation, decrease the contents of the by-product glutamic acid in the fermentation broth in the fermentation for L-lysine and direct more carbon flux to L-lysine synthesis to improve the utilization rate of the fermentation substrate of L-lysine and facilitate purification.

### EXPLANATION OF TERMS

In the present invention, the "inactivation" can be induced by any known inactivation method in this field. The effect of "inactivation" refers to when the expression of the endogenous gene NCgl1221 is reduced to a very low level or a gene which cannot be expressed is produced or the gene is expressed to produce a product which does not have any activity or whose activity is reduced.

In the present invention, "inactivation" may be done by inserting one or more base pairs into the endogenous gene NCgl1221, deleting one or more base pairs in the endogenous gene NCgl1221 or converting or reversing the internal sequence of the gene by means of genetic engineering.

The technical solution of the present invention is described below:
An engineered Corynebacterium glutamicum bacterium used for a high production of L-lysine is constructed by inactivating the gene NCgl1221 of Corynebacterium glutamicum and the nucleotide sequence of the NCgl1221 gene encoding frame is shown as set forth in SEQ ID NO. 1.

Preferably, the Corynebacterium glutamicum is taken from the China Center of Industrial Culture Collection (CICC) and its culture number is CICC 23604 according to the present invention.

Preferably, the construction method above includes the following steps according to the present invention:
(1) A gene segment greater than 400bp in length in the NCgl1221 gene encoding frame is amplified to be a homologous arm sequence through PCR and the nucleotide sequence of the NCgl1221 gene encoding frame is shown as set forth in SEQ ID NO. 1;
(2) A resistant marker gene segment is amplified through PCR;
(3) The homologous arm sequence obtained in Step (1) and the resistant marker gene sequence obtained in Step (2) are linked together through an overlap PCR to obtain a fused segment with both ends having the same restriction enzyme cutting sites and the enzyme cutting sites may not be in the proposed knock-out gene and the resistant marker gene;
(4) The competent cell of Corynebacterium glutamicum is prepared, and the fused gene obtained in Step (3) is digested and then the competent cell of Corynebacterium glutamicum is transformed.

Further preferably, the genome DNA of Corynebacterium glutamicum is used as a template to obtain the homologous arm NCg1 through PCR amplification in Step (1) according to the present invention, and the nucleotide sequence of the PCR amplification primers is shown as set forth below:
F1: CGGGATCCCGTTTTTCATGCTTGCCGTCT;
R1: AAGGCCAGCAAAA GCTAAAGCCCAAGAATGCAAC;

The reaction system of the PCR amplification is shown below, and the overall system is 50µl:

| | |
|---|---|
| 2×HiFi-PCR master | 25µl |
| F1 (10µmol/L) | 2µl |
| R1 (10µmol/L) | 2µl |
| Template | 2µl |
| dd H₂O | 19µl |

The PCR amplification procedure is described below:
Initial denaturalization for 5min at 95 °C, denaturalization for 30s at 94°C, annealing for 30s at 55°C, extension for 30s at 72°C, and 30 cycles; extension for 10min at 72 °C, and storage at 4°C.

Preferably, the PCR amplification plate in Step (2) is the DNA of the shuttle plasmid pHT01 (bought from Hangzhou Boi SCI Co., Ltd.) to obtain the resistant gene Cm^{r} of chloramphenicol according to the present invention, and the nucleotide sequence of the PCR amplification primers is shown below:
F2: GGTGGTCGGCATTTTTGCTGGCCTTTTGCTCA;
R2: CATAATCGGCTGGATCCTAGTGACTGGCGATGCT;

The reaction system of the PCR amplification is shown below, and the overall system is 50µl:

| | |
|---|---|
| 2×HiFi-PCR master | 25µl |
| F2 (10µMOl/L) | 2µl |
| R2 (10µmol/L) | 2µl |
| Template | 2µl |
| dd H₂O | 19µl |

The said PCR amplification procedure is described below:
Initial denaturalization for 5min at 95°C, denaturalization for 30s at 94°C, annealing for 30s at 55°C, extension for 3min at 72°C, and 30 cycles; extension for 10min at 72 °C, and storage at 4°C.

Preferably, the amplification system in the first phase of the overlap PCR in Step (3) is shown below according to the present invention, and the overall system is 25 µl:

| | |
|---|---|
| 2×HiFi-PCR master | 12.5µl |
| NCg1 | 2µl |
| Cm^{r} | 2µl |
| dd H₂O | 8.5µl |

The amplification procedure in the first phase of the overlap PCR is described below:
Initial denaturalization for 5min at 95°C, denaturalization for 30s at 94°C, annealing for 30s at 55°C, extension for 2min at 72°C, and 5 cycles; extension for 10min at 72 °C;
The amplification system in the second phase of the overlap PCR is prepared by adding the following constituents into the products obtained after amplification in the first phase, and the overall system is 50µl;

| | |
|---|---|
| 2xHiFi-PCR master | 12.5µl |
| F1 (10µmol/L) | 2µl |
| R2 (10µmol/L) | 2µl |
| dd H₂O | 8.5µl |

The amplification procedure in the second phase of the overlap PCR is described below.

Initial denaturalization for 5min at 95°C, denaturalization for 30s at 94°C, annealing for 30s at 58°C, extension for 4min at 72°C, and 30 cycles; extension for 10min at 72 °C, and storage at 4°C.

Preferably, the specific steps of Step (4) are described below according to the present invention:
(i) Select a single colony of Corynebacterium glutamicum, culture in a seed culture medium to a cell concentration OD₆₀₀ of 0.7∼0.9, cool with ice, centrifuge after cooling, wash the bacterial cells 3-5 times with a pre-cooled electrotransformation buffer and re-suspend the bacterial cells with the electrotransformation buffer to obtain competent cells;
(ii) Subject the fused gene obtained in Step (3) to single enzyme digestion at 28∼32 °C for 2∼5h, introduce into the competent cell obtained in Step (i) through high voltage electrotransformation, culture in a liquid resuscitation culture medium at 28 ∼32°C for 3∼4h, and conduct screening.

Further preferably, the single enzyme digestion system in Step (ii) is shown below according to the present invention, and the overall system is 40 µl:

| | |
|---|---|
| 10×K Buffer | 4µl |
| Restriction enzyme BamH I | 4µl |
| NCg1-Cm^{r} (Fused gene) | 20µl |
| dd H₂O | 12µl. |

Further preferably, the seed culture medium in Step (i) has the following constituents per liter according to the present invention:
Peptone 8∼12g, yeast powder 4∼6g, sodium chloride 8~12g, and sorbitol 85∼96 g.

Further preferably, the electrotransformation buffer in Step (i) has the following constituents per liter according to the present invention:
Sorbitol 85∼96 g, mannitol 85∼96 g and glycerol 95∼105 mL.

Further preferably, the conditions for high voltage electrotransformation in Step (i) are: electroporation at 2100V for 5ms according to the present invention.

Further preferably, the liquid resuscitation culture medium in Step (ii) has the following constituents per liter according to the present invention:
Peptone 8~12g, yeast powder 4~6g, sodium chloride 8~12g, sorbitol 85∼96 g and mannitol 65∼73g.

Application of the engineered Corynebacterium glutamicum bacteria having a high production of L-lysine obtained with the construction method above in production of L-lysine.

### PRINCIPLE OF CONSTRUCTION

The protein for transporting glutamic acid out of cells is responsible for the extracellular secretion of glutamic acid. However, the inventor finds that in bacteria with a high production of L-lysine through research, the expression of this gene increases the contents of the by-product glutamic acid in the fermentation broth in the fermentation for L-lysine and direct less carbon flux to L-lysine synthesis which will affect other metabolic processes of cells including lysine synthesis. Therefore, recombinant bacteria with a high production of L-lysine are obtained as a result of the inactivation of the gene NCgl1221 through the construction.

### BENEFICIAL EFFECTS

The present invention provides an application of the constructed Corynebacterium glutamicum bacterium strains for a high production of L-lysine. The extracellular secretion amount of glutamic acid is obviously reduced for the inactivation of the endogenous gene NCgl122 of the bacterium strain constructed. Compared with wild bacterial strains, the strain can produce a higher concentration of L-lysine, effectively reduce the contents of the by-product glutamic acid in the fermentation broth, and further reduce production and purification costs as the production strain of L-lysine.

### DETAILED DESCRIPTION OF THE INVENTION

Embodiment 1: construction of knock-out gene segment
1)
(i) Extract the genome DNA of Corynebacterium glutamicum 23604; use the genome DNA as the template for PCR amplification to obtain the homologous arm NCg1;
The said PCR primer sequences are shown as set forth below:
F1: CGGGATCCCGTTTTTCATGCTTGCCGTCT;
R1: AAGGCCAGCAAAAGCTAAAGCCCAAGAATGCAAC;
The said PCR amplification system is shown below:

| Reagent | Volume (µL) |
|---|---|
| 2×HiFi-PCR master | 25 |
| F1 (10µmol/L) | 2 |
| R1 (10µmol/L) | 2 |
| Template | 2 |
| dd H₂O | 19 |

The said PCR amplification procedure is described below:
Initial denaturalization for 5min at 95°C, denaturalization for 30s at 94°C, annealing for 30s at 55°C, extension for 30s at 72°C, and 30 cycles; extension for 10min at 72 °C, and storage at 4°C;
Test PCR products by agarose gel electrophoresis, and the length is 528bp. Use the SanPrep column DNA gel extraction kit (Shanghai Sangon Biotech) to recover the gel, and store recovered products at -20°C for later use;

(ii) Extract the DNA of the shuttle plasmid pHT01 (bought from Hangzhou Boi SCI Co., Ltd.), and use the DNA as the template for PCR amplification to obtain a Cm^{r} segment;
The said PCR primer sequences are shown as set forth below:
F₂: GGTGGTCGGCATTTTTGCTGGCCTTTTGCTCA;
R₂: CATAATCGGCTGGATCCTAGTGACTGGCGATGCT;
The said PCR amplification system is shown below:

| Reagent | Volume (µL) |
|---|---|
| 2×HiFi-PCR master | 25 |
| F₂ (10µmol/L) | 2 |
| R₂ (10µmol/L) | 2 |
| Template | 2 |
| dd H₂O | 19 |

The said PCR amplification procedure is described below:
Initial denaturalization for 5min at 95 °C, denaturalization for 30s at 94°C, annealing for 30s at 55 °C, extension for 3min at 72 °C, and 30 cycles; extension for 10min at 72 °C, and storage at 4 °C;
Test PCR products by agarose gel electrophoresis, and the length is 1274bp. Use the SanPrep column DNA gel extraction kit (Shanghai Sangon Biotech) to recover the gel, and store recovered products at -20°C for later use;

(iii) Subject the NCg1 segment obtained in Step (i) and the Cm^{r} segment obtained in Step (ii) to overlap PCR to obtain an NCg1-Cm^{r} segment;
The said overlap PCR amplification system is shown below:

| Reagent | Volume (µL) |
|---|---|
| (1) 2×HiFi-PCR master | 12.5 |
| NCg1 | 2 |
| Cm^{r} | 2 |
| dd H₂O | 8.5 |
| (2) Products after the first phase of amplification | 25 |
| 2×HiFi-PCR master | 12.5 |
| F1 (10µmol/L) | 2 |
| R2 (10µmol/L) | 2 |
| dd H₂O | 8.5 |

The amplification procedure in the first phase of the said overlap PCR is described below:
Initial denaturalization for 5min at 95°C, denaturalization for 30s at 94°C, annealing for 30s at 55°C, extension for 2min at 72°C, and 5 cycles; extension for 10min at 72 °C.
The amplification procedure in the second phase of the said overlap PCR is described below:
Initial denaturalization for 5min at 95 °C, denaturalization for 30s at 94°C, annealing for 30s at 58 °C, extension for 4min at 72 °C, and 30 cycles; extension for 10min at 72 °C, and storage at 4 °C;
Test PCR products by agarose gel electrophoresis, and the length is 1777bp. Use the SanPrep column DNA gel extraction kit (Shanghai Sangon Biotech) to recover the gel, and store recovered products at -20°C for later use;

Embodiment 2: preparation of competent bacillus licheniformis
(i) Pick a single colony of Corynebacterium glutamicum 23604, and introduce it into 10mL seed culture medium for overnight culture at 37°C and 220r/min;
   The seed culture medium has the following components:
   Peptone 10g, yeast powder 5g, sodium chloride 10g, and sorbitol 91g.
(ii) Take 1 mL of the bacteria solution above, introduce into 100 mL seed culture medium, and culture at 37°C and 220r/min to OD₆₀₀=0.9;
(iii) Transfer the bacteria solution to a 100 mL centrifuge tube, and allow the tube in a water bath for 15 - 20 min to stop the growth of bacterial cells;
(iv) After ice bathing, centrifuge 5000g at 4 °C for 5min to collect the bacterial cells;
(v) Wash centrifuged bacterial cells 3 times with a pre-cooled electrotransformation (ETM) buffer;
   The electrotransformation buffer has the following constituents per liter:
   sorbitol 96 g, mannitol 91 g and glycerol 100 mL.
(vi) After washing is done, re-suspend the bacterial cells with 1000 µL of electrotransformation buffer;
(vii) Load prepared competent cells into tubes by 100µL/tube, and store at -80°C for later use.

Embodiment 3: Introduction of an NCg1-Cm^{r} segment into Corynebacterium glutamicum 23604 through electrotransformation
(i) Digest the NCg1-Cm^{r} segment with the restriction enzyme BamH I at 30°C for 3h;
The enzyme digestion system (40µL) is shown below:

| Reagent | Volume (µL) |
|---|---|
| 10×K Buffer | 4 |
| BamH I | 4 |
| NCg1-Cm^{r} | 20 |
| dd H₂O | 12 |

(ii) Concentration and purification of enzyme-digested products
Add 1/10 volume of 3M sodium acetate and a volume of absolute ethyl alcohol that is 2.5 times that of the 3M sodium acetate and allow the solution to be in a -20°C refrigerator for 20min;
Centrifuge at 12000r/min for 5min to obtain precipitates;
Re-suspend the precipitates with 300 µL of 75 % ethyl alcohol (per cent by volume);
Centrifuge at 12000r/min for 5min to remove the ethyl alcohol and air dry at 37°C for 30 min;
Add 15∼18 µL of dd H₂O to re-suspend DNA, and store at -20°C.
(iii) Electrotransformation
First, use an ultra-micro spectrophotometer for nucleic acid to determine the concentration of NCg1-Cm^{r} segments, and when the concentration reaches up to 300µg/mL, conduct electroproration at 2100V for 5ms to realize electrotransformation, culture obtained cells in a resuscitation culture medium at 30 °C for 1h, take 100µL and spread on a LB solid culture medium containing 200µg/mL chloramphenicol, culture at 37°C for 2 days, and screen for transformants having chloramphenicol resistance.
The liquid resuscitation culture medium has the following constituents per liter:
Peptone 10g, yeast powder 5g, sodium chloride 10g, sorbitol 91g, and mannitol 69.4 g.
Embodiment 4: culture and identification of positive recombinant bacteria Pick the positive recombinant bacterial colonies mentioned above, and introduce into a liquid LB culture medium containing 200µg/mL chloramphenicol resistance for overnight culture at 37 °C. After the culture is done, use the kit supplied by Shanghai Sangon Biotech Co., Ltd. to extract recombinant DNA. Conduct amplification with the genome obtained as the template and F₁ and R₂ as the primers, and verify amplification products by agarose gel electrophoresis;
The said PCR primer sequences are shown as set forth below:
F₁: CGGGATCCCGTTTTTCATGCTTGCCGTCT
R₂: CATAATCGGCTGGATCCTAGTGACTGGCGATGCT

Where, those underlined are enzyme cutting sites
The PCR amplification system is 20µl:

| Reagent | Volume (µL) |
|---|---|
| 2×HiFi-PCR master | 10 |
| F₁ (10µmol/L) | 1 |
| R₂ (10µmol/L) | 1 |
| Template | 1 |
| dd H₂O | 7 |

The said PCR amplification procedure is described below:
Initial denaturalization for 5min at 95 °C, denaturalization for 30s at 94°C, annealing for 30s at 57°C, extension for 4min at 72°C, and 30 cycles; extension for 10min at 72 °C, and storage at 4°C;
Testing of PCR products by agarose gel electrophoresis shows that a specific gene band is amplified with the primers F₁ and R₂, and the size is around 1800b, close to the theoretical value 1777bp. This indicates that the target gene has been successfully integrated into the genome of the Corynebacterium glutamicum and engineered Corynebacterium glutamicum bacteria with a high production of L-lysine are obtained.

Embodiment 4: testing of fermentation for L-lysine
Introduce the engineered Corynebacterium glutamicum bacteria having a high production of L-lysine obtained into 100mL LBG culture medium (glucose 5g/L, peptone 10g/L, yeast extract 5g/L and NaCl 10g/L) for 20h seed culture at 220rpm and 30°C, and then introduce 2% (percent by volume) to 100 mL fermentation medium (glucose 100g/L, peptone 20g/L, corn steep liquor 30 mL, urea 5 g/L, (NH₄)₂SO₄ 25g/L, L-leucine 0.34g/L, KH₂PO₄ 2g/L, MgSO₄·7H₂O 1.5g/L and biotin 0.001g/L) for 36h fermentation culture. Use a Hitachi L-8900 model high-speed automatic amino acid analyzer to determine the amino acids contents in the fermentation broth.

The result shows that compared with the original bacteria, the glutamic acid content in the fermentation broth of engineered Corynebacterium glutamicum bacteria with a high production of L-lysine is reduced from 3.8 g/L to a level undetectable by instruments and the production of lysine is increased from 40.0 g/L to 43.2g/L.

## Claims

1. The application of an engineered Corynebacterium glutamicum bacteria having a high production of L-lysine constructed by inactivating the gene NCgl1221 of Corynebacterium glutamicum and having the nucleotide sequence of the NCgl1221 gene encoding frame as set forth in SEQ ID NO.1 in production of L-lysine.

2. The application as claimed in Claim 1, **characterized in that** Corynebacterium glutamicum is taken from (China Center of Industrial Culture Collection (CICC) and its culture number is CICC 23604.

3. The application as claimed in Claim 1 or 2, **characterized in that** Corynebacterium glutamicum is constructed including the following steps:
(1) A gene segment greater than 400bp in length in the NCg 11221 gene encoding frame is amplified to be as a homologous arm sequence through PCR, and the nucleotide sequence of the NCgl1221 gene encoding frame is shown as set forth in SEQ ID NO. 1;
(2) A resistant marker gene segment is amplified through PCR;
(3) the homologous arm sequence obtained in Step (1) and the resistant marker sequence obtained in Step (2) are linked together through PCR to obtain a fused segment with both ends having the same restriction enzyme cutting sites, and the enzyme cutting sites may not be in the proposed knock-out gene and the resistant marker gene;
(4) The competent cell of Corynebacterium glutamicum is prepared, and the fused gene obtained in Step (3) is digested and then the competent cell of Corynebacterium glutamicum is transformed.

4. The application as claimed in Claim 3, **characterized in that** the genome DNA of Corynebacterium glutamicum is used as a template for PCR amplification in Step (1), and the nucleotide sequence of the PCR amplification primers is shown as set forth below:
F1: CGGGATCCCGTTTTTCATGCTTGCCGTCT;
R1: AAGGCCAGCAAAAGCTAAAGCCCAAGAATGCAAC;
The reaction system of the PCR amplification is shown below, and the overall system is 50µl:
| | |
|---|---|
| 2×HiFi-PCR master | 25µl |
| F1 (10µmol/L) | 2µl |
| R1 (10µmol/L) | 2µl |
| Template | 2µl |
| dd H₂O | 19µl; |
The PCR amplification procedure is described below:
Initial denaturalization for 5min at 95 °C, denaturalization for 30s at 94°C, annealing for 30s at 54 °C, extension for 30s at 72 °C, and 30 cycles; extension for 10min at 72 °C, and storage at 4 °C.

5. The application as claimed in Claim 3, **characterized in that** the PCR amplification plate is the DNA of the shuttle plasmid pHT01 in Step (2), and the nucleotide sequence of the PCR amplification primers is shown as set forth below:
F2: GGTGGTCGGCATTTTTGCTGGCCTTTTGCTCA;
R2: CATAATCGGCTGGATCCTAGTGACTGGCGATGCT;
The reaction system of the PCR amplification is shown below, and the overall system is 50µl:
| | |
|---|---|
| 2×HiFi-PCR master | 25µl |
| F2 (10µmol/L) | 2µl |
| R2 (10µmol/L) | 2µl |
| Template | 2µl |
| dd H₂O | 19µl; |
The said PCR amplification procedure is described below:
Initial denaturalization for 5min at 95°C, denaturalization for 30s at 94°C, annealing for 30s at 55°C, extension for 3min at 72°C, and 30 cycles; extension for 10min at 72°C, and storage at 4°C.

6. The application as claimed in Claim 3, **characterized in that** the amplification system in the first phase of the overlap PCR in Step (3) is shown below, and the overall system is 25 µl:
| | |
|---|---|
| 2xHiFi-PCR master | 12.5µl |
| NCg1 | 2µl |
| Cm^{r} | 2µl |
| dd H₂O | 8.5µl |
The amplification procedure in the first phase of the overlap PCR is described below Initial denaturalization for 5min at 95°C, denaturalization for 30s at 94°C, annealing for 30s at 55°C, extension for 2min at 72°C, and 5 cycles; extension for 10min at 72 °C;
The amplification system in the second phase of the overlap PCR is prepared by adding the following constituents into the products obtained after amplification in the first phase, and the overall system is 50µl;
| | |
|---|---|
| 2×HiFi-PCR master | 12.5µl |
| F1 (10µmol/L) | 2µl |
| R2 (10µmol/L) | 2µl |
| dd H₂O | 8.5µl |
The amplification procedure in the second phase of the overlap PCR is described below:
Initial denaturalization for 5min at 95°C, denaturalization for 30s at 94°C, annealing for 30s at 58°C, extension for 4min at 72°C, and 30 cycles; extension for 10min at 72°C, and storage at 4°C.

7. The application as claimed in Claim 3, **characterized in that** the specific steps of Step (4) is described below:
(i) Select a single colony of Corynebacterium glutamicum, culture in a seed culture medium to a cell concentration OD₆₀₀ of 0.7∼0.9, cool with ice, centrifuge after cooling, wash the bacterial cells 3-5 times with a pre-cooled electrotransformation buffer, and re-suspend the bacterial cells with the electrotransformation buffer to obtain competent cells;
(ii) Subject the fused gene obtained in Step (3) to single enzyme digestion at 28∼32°C for 2∼5h, introduce into the competent cell obtained in Step (i) through high voltage electrotransformation, culture in a liquid resuscitation culture medium at 28∼32°C for 3∼4h, and conduct screening.

8. The application as claimed in Claim 7, **characterized in that** the single enzyme system in Step (ii) is shown below, and the overall system is 40 µl:
| | |
|---|---|
| 10×K Buffer | 4µl |
| Restriction enzyme BamHI | 4µl |
| NCg1-Cm^{r} | 20µl |
| dd H₂O | 12µl. |

9. The application as claimed in Claim 7, **characterized in that** the seed culture medium in Step (i) has the following constituents per liter:
Peptone 8∼12g, yeast powder 4~6g, sodium chloride 8∼12g, and sorbitol 85∼96 g;
Preferably, the electrotransformation buffer in Step (i) has the following constituents per liter:
Sorbitol 85∼96 g, mannitol 85∼96 g and glycerol 95∼105 mL;
Preferably, the conditions for high voltage electrotransformation in Step (i) are: electroporation at 2100V for 5ms;
Preferably, the liquid resuscitation culture medium in Step (ii) has the following constituents per liter:
Peptone 8∼12g, yeast powder 4~6g, sodium chloride 8∼12g, sorbitol 85∼96 g and mannitol 65∼73g.

## Patentansprüche

1. Verwendung eines manipulierten Corynebacterium glutamicum-Bakteriums mit einer hohen L-Lysin-Produktion, das durch Inaktivierung des Gens NCgl1221 von Corynebacterium glutamicum konstruiert wird und die Nukleotidsequenz des NCgl1221-Gen-Kodierrahmens aufweist, wie in SEQ ID NO. 1 dargestellt, bei der Herstellung von L-Lysin.

2. Verwendung nach Anspruch 1, **dadurch gekennzeichnet, dass** Corynebacterium glutamicum vom (China Center of Industrial Culture Collection (CICC)) entnommen wird und seine Kulturnummer CICC 23604 ist.

3. Verwendung nach Anspruch 1 oder 2, **dadurch gekennzeichnet, dass** Corynebacterium glutamicum konstruiert wird, umfassend die folgenden Schritte:
(1) ein Gensegment mit einer Länge von mehr als 400 bp im NCgl1221-Gen-Kodierrahmen wird durch PCR zu einer homologen Armsequenz amplifiziert, und die Nukleotidsequenz des NCgl1221-Gen-Kodierrahmens wird gezeigt wie in SEQ ID NO. 1 dargestellt;
(2) ein resistentes Markergensegment wird durch PCR amplifiziert;
(3) die in Schritt (1) erhaltene homologe Armsequenz und die in Schritt (2) erhaltene resistente Markersequenz werden durch PCR miteinander fusioniert, um ein fusioniertes Segment zu erhalten, wobei beide Enden die gleichen Restriktionsenzym-Schnittstellen aufweisen, und die Enzym-Schnittstellen dürfen nicht im vorgeschlagenen Knock-out-Gen und im resistenten Markergen liegen;
(4) die kompetente Zelle von Corynebacterium glutamicum wird hergestellt, und das in Schritt (3) erhaltene fusionierte Gen wird verdaut und dann wird die kompetente Zelle von Corynebacterium glutamicum transformiert.

4. Verwendung nach Anspruch 3, **dadurch gekennzeichnet, dass** die Genom-DNA von Corynebacterium glutamicum als Matrize für die PCR-Amplifikation in Schritt (1) verwendet wird, und die Nukleotidsequenz der PCR-Amplifikationsprimer gezeigt ist wie im Folgenden dargestellt:
F1: CGGGATCCCGTTTTTCATGCTTGCCGTCT;
R1: AAGGCCAGCAAAAGCTAAAGCCCAAGAATGCAAC;
das Reaktionssystem der PCR-Amplifikation ist im Folgenden dargestellt, und das Gesamtsystem beträgt 50 µl:
| | |
|---|---|
| 2 x HiFi-PCR-Master | 25 µl |
| F1 (10 µmol/l) | 2 µl |
| R1 (10 µmol/l) | 2 µl |
| Matrize | 2 µl |
dd H₂O (Aqua bidestillata) 19 µl;
das PCR-Amplifikationsverfahren wird im Folgenden beschrieben:
anfängliche Denaturierung für 5 Minuten bei 95 °C, Denaturierung für 30 s bei 94 °C, Annealing für 30 s bei 54 °C, Elongation für 30 s bei 72 °C und 30 Zyklen; Elongation für 10 min bei 72 °C und Lagerung bei 4 °C.

5. Verwendung nach Anspruch 3, **dadurch gekennzeichnet, dass** die PCR-Amplifikationsplatte die DNA des Shuttle-Plasmids pHT01 in Schritt (2) ist, und die Nukleotidsequenz der PCR-Amplifikationsprimer gezeigt ist wie im Folgenden dargestellt:
F2: GGTGGTCGGCATTTTTGCTGGCCTTTTGCTCA;
R2: CATAATCGGCTGGATCCTAGTGACTGGCGATGCT:
das Reaktionssystem der PCR-Amplifikation ist im Folgenden dargestellt, und das Gesamtsystem beträgt 50 µl:
| | |
|---|---|
| 2 x HiFi-PCR-Master | 25 µl |
| F2 (10 µmol/l) | 2 µl |
| R2 (10 µmol/l) | 2 µl |
| Matrize | 2 µl |
| dd H₂O | 19 µl |
dieses PCR-Amplifikationsverfahren wird im Folgenden beschrieben:
anfängliche Denaturierung für 5 Minuten bei 95 °C, Denaturierung für 30 s bei 94 °C, Annealing für 30 s bei 55 °C, Elongation für 3 min bei 72 °C und 30 Zyklen; Elongation für 10 min bei 72 °C und Lagerung bei 4 °C.

6. Verwendung nach Anspruch 3, **dadurch gekennzeichnet, dass** das Amplifikationssystem in der ersten Phase der Überlappungs-PCR in Schritt (3) im Folgenden dargestellt ist und das Gesamtsystem 25 µl beträgt:
| | |
|---|---|
| 2 x HiFi-PCR-Master | 12,5 µl |
| NCgl | 2 µl |
| Cm^{r} | 2 µl |
| dd H₂O | 8,5 µ1 |
das Amplifikationsverfahren in der ersten Phase der Überlappungs-PCR wird im Folgenden beschrieben
anfängliche Denaturierung für 5 Minuten bei 95 °C, Denaturierung für 30 s bei 94 °C, Annealing für 30 s bei 55 °C, Elongation für 2 min bei 72 °C und 5 Zyklen; Elongation für 10 min bei 72 °C;
das Amplifikationssystem in der zweiten Phase der Überlappungs-PCR wird durch Zugabe der folgenden Bestandteile zu den nach der Amplifikation in der ersten Phase erhaltenen Produkten hergestellt, wobei das Gesamtsystem 50 µl beträgt;
| | |
|---|---|
| 2 x HiFi-PCR-Master | 12,5 µl |
| F1 (10 µmol/l) | 2 µl |
| R2 (10 µmol/l) | 2 µl |
| dd H₂O | 8,5 µl |
das Amplifikationsverfahren in der zweiten Phase der Überlappungs-PCR wird im Folgenden beschrieben
anfängliche Denaturierung für 5 Minuten bei 95 °C, Denaturierung für 30 s bei 94 °C, Annealing für 30 s bei 58 °C, Elongation für 4 min bei 72 °C und 30 Zyklen; Elongation für 10 min bei 72 °C und Lagerung bei 4 °C.

7. Verwendung nach Anspruch 3, **dadurch gekennzeichnet, dass** die spezifischen Schritte von Schritt (4) im Folgenden beschrieben werden:
(i) Auswählen einer einzelnen Kolonie von Corynebacterium glutamicum, Kultivieren in einem Impfkulturmedium auf eine Zellkonzentration OD₆₀₀ von 0,7 ∼0,9, Kühlen mit Eis, Zentrifugieren nach dem Abkühlen, Waschen der Bakterienzellen 3 ∼ 5 Mal mit einem vorgekühlten Elektrotransformationspuffer, und Resuspendieren der Bakterienzellen mit dem Elektrotransformationspuffer, um kompetente Zellen zu erhalten;
(ii) enzymatisches Einzelverdauen des in Schritt (3) erhaltene fusionierten Gens bei 28 ∼ 32 °C für 2 ∼ 5 h, Einführen in die in Schritt (i) erhaltene kompetente Zelle durch Hochspannungs-Elektrotransformation, Kultivieren in einem flüssigen Rückgewinnungs-Kulturmedium bei 28 ∼ 32 °C für 3 ∼ 4 h und Durchführen eines Screenings.

8. Verwendung nach Anspruch 7, **dadurch gekennzeichnet, dass** das enzymatische Einzelsystem in Schritt (ii) im Folgenden dargestellt ist und das Gesamtsystem 40 µl beträgt:
| | |
|---|---|
| 10xK Puffer | 4 µl |
| Restriktionsenzym BamHI | 4 µl |
| NCgl-Cm^{r} | 20 µl |
| dd H₂O | 12 µl. |

9. Verwendung nach Anspruch 7, **dadurch gekennzeichnet, dass** das Impfkulturmedium in Schritt (i) die folgenden Bestandteile pro Liter aufweist:
Pepton 8 ∼ 12 g, Hefepulver 4 ∼ 6 g, Natriumchlorid 8 ∼ 12 g und Sorbitol 85 ∼ 96 g;
vorzugsweise weist der Elektrotransformationspuffer in Schritt (i) die folgenden Bestandteile pro Liter auf:
Sorbitol 85 ∼ 96 g, Mannitol 85 ∼ 96 g und Glycerol 95 ∼ 105 ml;
vorzugsweise sind die Bedingungen für die Hochspannungs-Elektrotransformation in Schritt (i):
Elektroporation bei 2.100 V für 5 ms;
vorzugsweise hat das flüssige Rückgewinnungskulturmedium in Schritt (ii) die folgenden Bestandteile pro Liter:
Pepton 8 ∼ 12 g, Hefepulver 4 ∼ 6 g, Natriumchlorid 8 ∼ 12 g, Sorbitol 85 ∼ 96 g und Mannitol 65 ∼ 73 g.

## Revendications

1. Application d'une bactérie *Corynebacterium glutamicum* génétiquement modifiée ayant une production élevée de L-lysine construite par inactivation du gène NCgl1221 de *Corynebacterium glutamicum* et ayant la séquence nucléotidique du cadre de codage du gène NCgl1221 telle qu'exposée dans SEQ ID NO : 1 dans la production de L-lysine.

2. Application selon la revendication 1, **caractérisée en ce que** *Corynebacterium glutamicum* provient du CICC (China Center of Industrial Culture Collection) et son numéro de culture est CICC 23604.

3. Application selon la revendication 1 ou 2, **caractérisée en ce que** *Corynebacterium glutamicum* est construit avec les étapes suivantes :
(1) un segment génique d'une longueur supérieure à 400 pb dans le cadre de codage du gène NCgl1221 est amplifié pour être comme une séquence de bras homologue par PCR, et la séquence nucléotidique du cadre de codage du gène NCgl1221 est présentée telle qu'exposée dans SEQ ID NO : 1 ;
(2) un segment de gène marqueur de résistance est amplifié par PCR ;
(3) la séquence de bras homologue obtenue à l'étape (1) et la séquence de marqueur de résistance obtenue à l'étape (2) sont liées l'une à l'autre par PCR pour obtenir un segment fusionné avec les deux extrémités ayant les mêmes sites de coupure par des enzymes de restriction, et les sites de coupures enzymatiques peuvent n'être pas dans le gène inactivé proposé et le gène marqueur de résistance ;
(4) la cellule compétente de *Corynebacterium glutamicum* est préparée, et le gène fusionné obtenu à l'étape (3) est digéré puis la cellule compétente de *Corynebacterium glutamicum* est transformée.

4. Application selon la revendication 3, **caractérisée en ce que** l'ADN génomique de *Corynebacterium glutamicum* est utilisé en tant que matrice pour l'amplification par PCR à l'étape (1), et la séquence nucléotidique des amorces d'amplification par PCR est présentée telle qu'exposée ci-dessous :
F1 : CGGGATCCCGTTTTTCATGCTTGCCGTCT ;
R1: AAGGCCAGCAAAAGCTAAAGCCCAAGAATGCAAC;
le système de réaction de l'amplification par PCR est présenté ci-dessous, et la totalité du système constitue 50 µl:
| | |
|---|---|
| 2 x HiFi-PCR master | 25 µl |
| F1 (10 µmol/l) | 2 µl |
| R1 (10 µmol/l) | 2 µl |
| Matrice | 2 µl |
| dd H₂O | 19 µl; |
la procédure d'amplification par PCR est décrite ci-dessous :
dénaturation initiale pendant 5 minutes à 95 °C, dénaturation pendant 30 s à 94 °C, hybridation pendant 30 s à 54 °C, extension pendant 30 s à 72 °C, et 30 cycles ; extension pendant 10 minutes à 72 °C, et stockage à 4 °C.

5. Application selon la revendication 3, **caractérisée en ce que** la plaque d'amplification par PCR est l'ADN du plasmide navette pHT01 à l'étape (2), et la séquence nucléotidique des amorces d'amplification par PCR est présentée telle qu'exposée ci-dessous :
F2 : GGTGGTCGGCATTTTTGCTGGCCTTTTGCTCA ;
R2 : CATAATCGGCTGGATCCTAGTGACTGGCGATGCT ;
le système de réaction de l'amplification par PCR est présenté ci-dessous, et la totalité du système constitue 50 µl:
| | |
|---|---|
| 2 x HiFi-PCR master | 25 µl |
| F2 (10 µmol/l) | 2 µl |
| R2 (10 µmol/l) | 2 µl |
| Matrice | 2 µl |
| dd H₂O | 19 µl ; |
ladite procédure d'amplification par PCR est décrite ci-dessous :
dénaturation initiale pendant 5 minutes à 95 °C, dénaturation pendant 30 s à 94 °C, hybridation pendant 30 s à 55 °C, extension pendant 3 minutes à 72 °C, et 30 cycles ; extension pendant 10 minutes à 72 °C, et stockage à 4 °C.

6. Application selon la revendication 3, **caractérisée en ce que** le système d'amplification dans la première phase de la PCR par chevauchement à l'étape (3) est présenté ci-dessous, et la totalité du système constitue 25 µl:
| | |
|---|---|
| 2 x HiFi-PCR master | 12,5 µl |
| NCg1 | 2 µl |
| Cm^{r} | 2 µl |
| dd H₂O | 8,5 µl |
la procédure d'amplification dans la première phase de la PCR par chevauchement est décrite ci-dessous
dénaturation initiale pendant 5 minutes à 95 °C, dénaturation pendant 30 s à 94 °C, hybridation pendant 30 s à 55 °C, extension pendant 2 minutes à 72 °C, et 5 cycles ; extension pendant 10 minutes à 72 °C ;
le système d'amplification dans la seconde phase de la PCR par chevauchement est préparé par ajout des constituants suivants dans les produits obtenus après l'amplification dans la première phase, et la totalité du système constitue 50 µl ;
| | |
|---|---|
| 2 x HiFi-PCR master | 12,5 µl |
| F1 (10 µmol/l) | 2 µl |
| R2 (10 µmol/l) | 2 µl |
| dd H₂O | 8,5 µl |
la procédure d'amplification dans la seconde phase de la PCR par chevauchement est décrite ci-dessous :
dénaturation initiale pendant 5 minutes à 95 °C, dénaturation pendant 30 s à 94 °C, hybridation pendant 30 s à 58 °C, extension pendant 4 minutes à 72 °C, et 30 cycles ; extension pendant 10 minutes à 72 °C, et stockage à 4 °C.

7. Application selon la revendication 3, **caractérisée en ce que** les étapes spécifiques de l'étape (4) sont décrites ci-dessous :
(i) sélection d'une colonie unique de *Corynebacterium glutamicum,* culture dans un milieu de culture à une concentration cellulaire DO₆₀₀ de 0,7 à 0,9, refroidissement avec de la glace, centrifugation après le refroidissement, lavage des cellules bactériennes 3 à 5 fois avec un tampon d'électrotransformation pré-refroidi, et remise en suspension des cellules bactériennes avec le tampon d'électrotransformation pour obtenir des cellules compétentes ;
(ii) soumission du gène fusionné obtenu à l'étape (3) à une digestion enzymatique unique à 28 à 32 °C pendant 2 à 5 h, introduction dans la cellule compétente obtenue à l'étape (i) par électrotransformation haute tension, culture dans un milieu de culture de ressuscitation liquide à 28 à 32 °C pendant 3 à 4 h, et réalisation d'un criblage.

8. Application selon la revendication 7, **caractérisée en ce que** le système enzymatique unique à l'étape (ii) est présenté ci-dessous, et la totalité du système constitue 40 µl:
| | |
|---|---|
| tampon 10 × K | 4 µl |
| enzyme de restriction BamH I | 4 µl |
| NCg1-Cm^{r} | 20 µl |
| dd H₂O | 12 µl. |

9. Application selon la revendication 7, **caractérisée en ce que** le milieu de culture d'ensemencement à l'étape (i) a les constituants suivants par litre :
peptone 8 à 12 g, poudre de levure 4 à 6 g, chlorure de sodium 8 à 12g, et sorbitol 85 à 96 g ;
de préférence, le tampon d'électrotransformation à l'étape (i) a les constituants suivants par litre :
sorbitol 85 à 96 g, mannitol 85 à 96 g et glycérol 95 à 105 ml ;
de préférence, les conditions d'électrotransformation haute tension à l'étape (i) sont :
électrotransformation à 2 100 V pendant 5 ms ;
de préférence, le milieu de culture de ressuscitation liquide à l'étape (ii) a les constituants suivants par litre :
peptone 8 à 12 g, poudre de levure 4 à 6 g, chlorure de sodium 8 à 12 g, sorbitol 85 à 96 g et mannitol 65 à 73 g.
